# EUROPÄISCHE PATENTSCHRIFT

(11) **EP 1 389 990 B1**
(45) Veröffentlichungstag und Bekanntmachung des Hinweises auf die Patenterteilung: **25.07.2007**
(21) Anmeldenummer: 02735313.5
(22) Anmeldetag: 27.04.2002
(51) Int. Cl.: A61F 9/02

(54) **Schutzbrille**
Safety glasses
Lunettes de protection

(30) Priorität: 30.05.2001 DE 20109028 U
(43) Veröffentlichungstag der Anmeldung: 25.02.2004
(73) Patentinhaber: Uvex Arbeitsschutz GmbH, 90766 Fürth (DE)
(72) Erfinder: WIEDNER, Klaus, 90768 Fürth (DE)
(74) Vertreter: Schneck, Herbert
(86) Internationale Anmeldenummer: PCT/EP2002/004685
(87) Internationale Veröffentlichungsnummer: WO 2002/096330

(56) Entgegenhaltungen:
- WO-A-95/25491
- WO-A-97/41815
- DE-B- 1 193 639
- FR-A- 2 302 718
- FR-A- 2 684 292
- US-A- 4 171 543
- US-A- 6 098 204

## Beschreibung

Die Erfindung richtet sich auf eine Schutzbrille, insbesondere Arbeitsschutzbrille, umfassend eine gewölbte Sichtscheibe mit seitlichen Scheibenenden und einen weichen Rahmenteil zwischen Scheibe und Gesichtsanlagebereich, wobei der Rahmenteil nach Art eines Faltenbalgs ausgebildet ist und wenigstens eine Faltung aufweist.

Eine Schutzbrille der gattungsgemäßen Art ist aus DE 11 93 639 B1 bekannt.

Hiervon ausgehend liegt der Erfindung die Aufgabe zugrunde, eine Schutzbrille zu schaffen, die eine optimale Anpassung des Rahmenteils und des Gesichtsanlagebereiches an die Anatomie des Benutzers und gleichzeitig hohen Tragekomfort und optimale Sicherheit ermöglicht, wobei insbesondere verhindert werden soll, dass Partikel oder Spritzer durch die Belüftungsöffnungen eindringen.

Diese Aufgabe wird erfindungsgemäß dadurch gelöst, dass die seitlichen Scheibenenden seitlich über den Rahmenteil überstehen und der Rahmenteil im Bereich der überstehenden Scheibenenden Belüftungsöffnungen aufweist.

Durch den erfindungsgemäßen Belüftungsspalt wird erreicht, dass einerseits kein direkter Weg zwischen Außenseite und Brilleninnenraum existiert und andererseits trotzdem eine gute Belüftung gegeben ist. Die Scheibe fungiert gleichzeitig als Abdeckung für die Belüftungsöffnungen.

Vorzugsweise sind auf die seitlichen Scheibenenden Kopfbandhaken aufrastbar. Diese Ausgestaltung ist sowohl in Verbindung mit einem faltenbalgartigen Rahmenteil als auch für sich genommen von großem Vorteil, weil ein leichter Scheibenaustausch realisierbar ist und die Scheibe selbst eine Art tragende Funktion übernehmen kann. An den Kopfbandhaken wird in an sich bekannter Weise ein Kopfband befestigt.

Diese Ausgestaltung eröffnet außerdem die einfache Handhabung durch Automaten bei der Herstellung.

Mit Vorteil ist der annähernd vertikal verlaufende seitliche Scheibenrand als Rastvorsprung zum Aufrasten der Kopfbandhaken ausgebildet, der mit einer korrespondierenden Rastausnehmung am Kopfbandhaken zusammenwirkt.

Im Bereich der Belüftungsöffnungen zwischen Rahmenteil und seitlichem Scheibenenden können Abstandshalter vorgesehen sein, die dafür sorgen, dass die Belüftungsöffnungen auch dann freigehalten werden, wenn die Scheibe aufgrund der Zugwirkung des Kopfbandes gegen das Rahmenteil gedrückt wird.

Zur Erzielung einer größeren Elastizität können die seitlichen Scheibenenden beiderseits des Rastvorsprungs geschlitzt sein.

Weiterhin können auch im Bereich der Oberkante und Unterkante der Scheibe zusätzliche Rastvorsprünge vorgesehen sein.

Nachfolgend wird die Erfindung anhand eines bevorzugten Ausführungsbeispiels in Verbindung mit der Zeichnung näher erläutert. Dabei zeigen:
- Fig. 1: eine perspektivische Ansicht einer erfindungsgemäßen Schutzbrille,
- Fig. 2: einen Schnitt längs der Linie II-II in Fig. 1,
- Fig. 3: eine Ansicht der Scheibe von vorne, wobei lediglich rechts ein Kopfbandhaken aufgerastet ist,
- Fig. 4: eine Draufsicht der in Fig. 3 dargestellten Scheibe,
- Fig. 5: einen Schnitt längs der Linie IV-IV in Fig. 3,
- Fig. 6: eine perspektivische Teil-Ansicht der Schutzbrille von innen und
- Fig. 7: eine Teil-Draufsicht auf den in Fig. 6 dargestellten Bereich der Schutzbrille.

Eine in der Zeichnung dargestellte Schutzbrille 1 umfaßt eine einstückige durchgehende gekrümmte Scheibe 2 und ein weiches Rahmenteil 3.

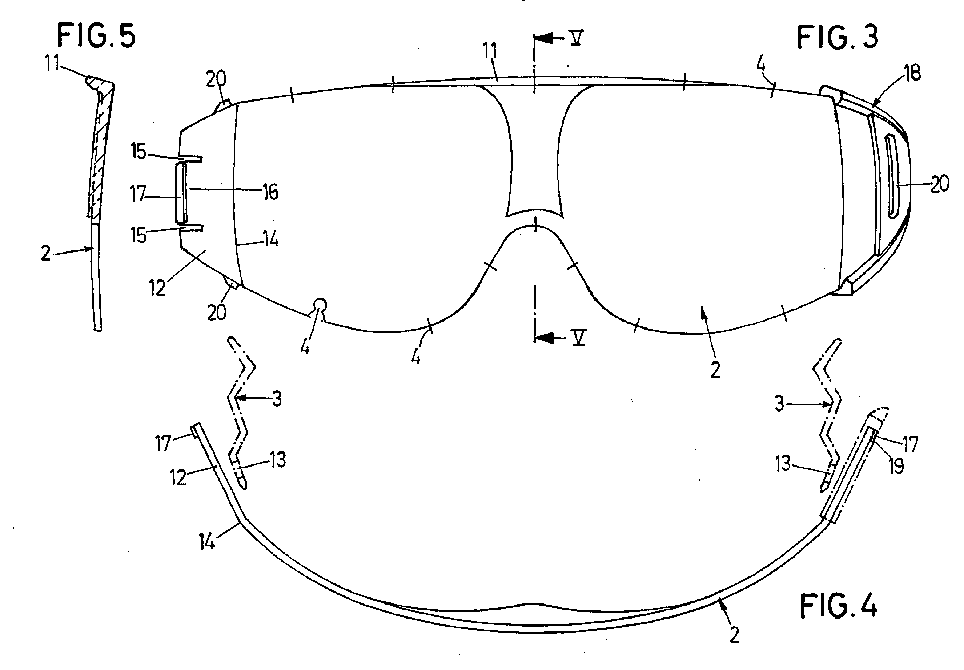

Die starre, eigenstabile Scheibe 2 trägt das Rahmenteil 3, wobei der Rahmenteil 3 in an sich bekannter Weise über sogenannte Schlüssellöcher 4 an der Scheibe festgelegt ist. In Fig. 3 ist eines dieser Schlüssellöcher gezeichnet, die anderen sind lediglich durch Striche angedeutet.

Der Rahmenteil 3 ist im Bereich der Oberkante 5 der Scheibe 2 mit dieser so verbunden, daß eine Luftdurchlaßöffnung 6 frei bleibt, die durch den herabgezogenen vorderen Außenrand 7 des Rahmenteils 3 abgedeckt ist.

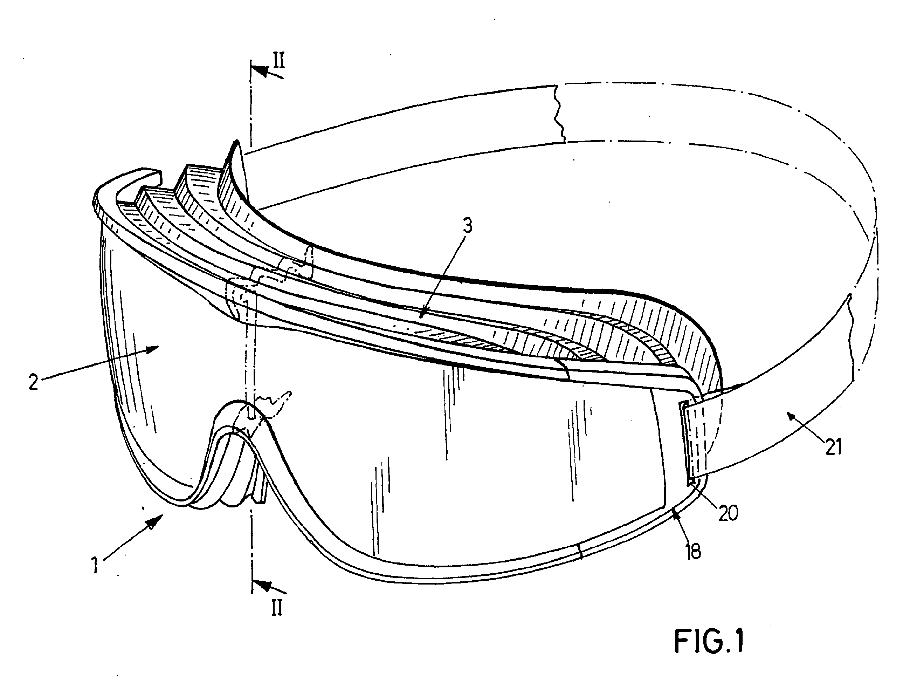

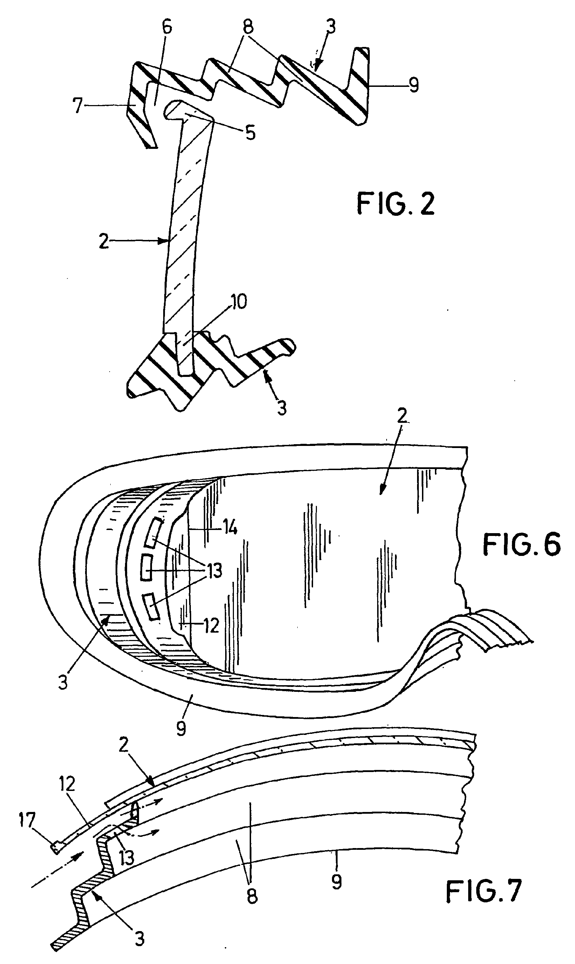

Der Rahmenteil 3 ist, wie insbesondere aus Fig. 1 und 2 erkennbar, als eine Art Faltenbalg ausgebildet und umfaßt dementsprechend mehrere Faltungen 8, wodurch eine große Elastizität und Anpaßbarkeit erreicht wird, so daß der Gesichtsanlagebereich 9 unabhängig von der spezifischen Anatomie des Benutzers dem Gesichts immer einerseits dicht und andererseits doch bequem anliegt.

Im Bereich des unteren Randes 10 der Scheibe 2 umgreift der Rahmenteil 3 die Scheibe 2 formschlüssig.

Wie aus Fig. 5 erkennbar ist, weist die Scheibe 2 an ihrer Oberseite einen nach vorne gerichteten Vorsprung 11 auf. Dieser Vorsprung 11 dient zur Halterung des Rahmenteils 3 und gleichzeitig als Abstandhalter zur Erzeugung eines Abstandes zwischen Oberseite der Scheibe 2 und Rahmenteil 3 zur Ausbildung einer Belüftungsöffnung. Um diesen Effekt noch zu verstärken, können zusätzliche noppenartige Abstandshalter im Bereich des Vorsprunges 11 und/oder eine oder mehrere Ausnehmungen in der Scheibe vorgesehen sein, die aber nach vorne hin in jedem Fall vom Rahmenteil überdeckt sind.

Der Rahmenteil 3 ist im Bereich der seitlichen Enden 12 mit Belüftungsöffnungen 13 versehen, die durch die gegenüber der gewölbten Scheibe bei 14 abgeknickten seitlichen Enden 12 abgedeckt werden, so daß durch die Belüftungsöffnungen 13 weder Strahlung noch Partikel oder Spritzer in den Innenbereich gelangen können.

In diesem Bereich sind an dem Rahmenteil noppenartige, in der Zeichnung nicht dargestellte, Abstandshalter vorgesehen, die verhindern, daß beim Auftreten einer Zugbelastung über das Kopfband auf die Scheibe die seitlichen Belüftungsöffnungen verschlossen werden.

Wie aus der linken Seite in Fig. 3 erkennbar ist, ist an den seitlichen Enden 12 durch zwei Schlitze 15 eine Federzunge 16 ausgebildet, welche im Bereich des vertikalen Außenrandes mit einem Rastvorsprung 17 versehen ist.

Auf die seitlichen Enden sind Kopfbandhaken 18 aufrastbar, wovon in Fig. 3 einer rechts dargestellt ist. Die Kopfbandhaken 18 weisen eine Rastausnehmung 19 auf, in die die Rastvorsprünge 17 einrasten. Zusätzlich können noch Vorsprünge 22 im Bereich der Oberkante und Unterkante der seitlichen Enden der Scheibe 2 vorgesehen sein, die in korrespondierende, in der Zeichnung nicht dargestellte Ausnehmungen der Kopfbandhaken 18 einrasten. Die Rastausnehmungen 19 sind vorteilhafterweise in Form einer Erweiterung (vgl. Fig. 3 rechts) der Schlitze 20 ausgebildet. Zum Auswechseln der Scheibe 2 braucht von außen lediglich auf die Rastvorsprünge 17 gedrückt zu werden, so daß diese mit den Rastausnehmungen 19 außer Eingriff kommen.

Die Kopfbandhaken 18 sind mit Schlitzen 20 versehen, durch welche ein Kopfband 21 geführt werden kann.

## Patentansprüche

1. Schutzbrille (1), insbesondere Arbeitsschutzbrille, umfassend eine gewölbte Sichtscheibe (2) mit seitlichen Scheibenenden (12) und einen weichen Rahmenteil (3) zwischen Scheibe (2) und Gesichtsanlagebereich (9), wobei der Rahmenteil (3) nach Art eines Faltenbalgs ausgebildet ist und wenigstens eine Faltung (8) aufweist, **dadurch gekennzeichnet, dass** die seitlichen Scheibenenden (12) seitlich über den Rahmenteil (3) überstehen und der Rahmenteil (3) im Bereich der überstehenden Scheibenenden (12) Belüftungsöffnungen (13) aufweist.

2. Schutzbrille (1) nach Anspruch 1, **dadurch gekennzeichnet, dass** die Faltung (8) bzw. Faltungen (8) um die Scheibe (2) umlaufend ausgebildet sind.

3. Schutzbrille (1) nach Anspruch 1, **dadurch gekennzeichnet, dass** der Rahmenteil (3) die Scheibenoberseite unter Freilassung eines Belüftungsspaltes übergreift.

4. Schutzbrille nach Anspruch 1, **dadurch gekennzeichnet, dass** im Bereich der Belüftungsöffnungen (13) zwischen Rahmenteil (3) und seitlichem Scheibenende (12) Abstandshalter zwischen Scheibe (2) und Rahmenteil (3) vorgesehen sind.

5. Schutzbrille (1) nach Anspruch 1, **dadurch gekennzeichnet, dass** auf die seitlichen Scheibenenden (12) Kopfbandhaken (18) aufrastbar sind.

6. Schutzbrille(1) nach Anspruch 5, **dadurch gekennzeichnet, dass** der annähernd vertikal verlaufende seitliche Scheibenrand (12) als Rastvorsprung (22) zum Aufrasten der Kopfbandhaken (18) ausgebildet ist.

7. Schutzbrille (1) nach Anspruch 6, **dadurch gekennzeichnet, dass** die seitlichen Scheibenenden (12) beiderseits des Rastvorsprungs (22) geschlitzt sind.

## Claims

1. A pair of safety goggles (1), in particular industrial safety goggles, comprising a curved sight piece (2) with lateral sight-piece ends (12) and a soft frame (3) between the sight piece (2) and face contact area (9), wherein the frame is designed in the way of a bellows frame (3), having at least one fold (8), **characterized in that** the lateral sight-piece ends (12) reach beyond the frame (3) laterally and the frame (3) has airing holes (13) in the vicinity of the overlapping sight-piece ends (12).

2. A pair of safety goggles (1) according to claim 1, **characterized in that** the fold (8) or folds (8) are designed to encircle the sight piece (2).

3. A pair of safety goggles (1) according to claim 1, **characterized in that** the frame (3) encompasses the top of the sight piece, leaving an airing gap.

4. A pair of safety goggles (1) according to claim 1, **characterized in that** in the vicinity of the airing holes (13) between the frame (3) and lateral sight-piece end (12), spacers are provided between the sight piece (2) and frame (3).

5. A pair of safety goggles (1) according to claim 1, **characterized in that** head strap clasps (18) are lockable into place on the lateral sight-piece ends (12).

6. A pair of safety goggles (1) according to claim 5, **characterized in that** the approximately vertical lateral edge of the sight piece (12) is designed as a locking projection (22) for snap-engagement with the head strap clasps (18).

7. A pair of safety goggles (1) according to claim 6, **characterized in that** the lateral sight-piece ends (12) are slit on either side of the locking projection (22).

## Revendications

1. Lunettes de protection (1), en particulier des lunettes de protection de travail, comportant une visière incurvée (2) avec des extrémités de visière latérales (12) et un cadre souple (3) entre la visière (2) et la zone d'application faciale (9), où le cadre (3) est conçu comme un soufflet et présente au moins un pli (8), **caractérisée en ce que** les extrémités de visière latérales (12) dépassent latéralement sur le cadre (3) et le cadre (3)présente des ouvertures d'aération (13) au niveau des extrémités de visière (12) dépassant.

2. Lunettes de protection (1) selon la revendication 1, **caractérisée en ce que** le pli (8) ou les plis (8) sont conçus de sorte qu'ils tombent sur la visière (2).

3. Lunettes de protection (1) selon la revendication 1, **caractérisée en ce que** le cadre (3) prend sur la partie supérieure de la visière en laissant une fente d'aération.

4. Lunettes de protection (1) selon la revendication 1, **caractérisée en ce que** des espaceurs sont prévus entre la visière (2) et le cadre (3) au niveau des ouvertures d'aération (13) entre le cadre (3) et les extrémités de visière latérales (12).

5. Lunettes de protection (1) selon la revendication 1, **caractérisée en ce que** sur les extrémités de visière latérales (12), des crochets à bandeau (18) peuvent être posés.

6. Lunettes de protection (1) selon la revendication 5, **caractérisée en ce que** le bord de visière latéral (12) à peu près vertical est conçu comme une butée d'arrêt (22) pour la pose des crochets à bandeau (18).

7. Lunettes de protection (1) selon la revendication 6, **caractérisée en ce que** les extrémités de visière latérales (12) sont fendues des deux côtés de la butée d'arrêt (22)
